# EUROPÄISCHE PATENTSCHRIFT

(11) **EP 3 426 142 B1**
(45) Veröffentlichungstag und Bekanntmachung des Hinweises auf die Patenterteilung: **13.05.2020**
(21) Anmeldenummer: 17711072.3
(22) Anmeldetag: 06.03.2017
(51) Int. Cl.: A61B 5/0478, A61B 5/00

(54) **ELEKTRODENHAUBE**
ELECTRODE CAP
CASQUE À ÉLECTRODES

(30) Priorität: 09.03.2016 AT 501982016
(43) Veröffentlichungstag der Anmeldung: 16.01.2019
(73) Patentinhaber: Guger, Christoph, 4533 Piberbach (AT); Edlinger, Günter, 8020 Graz (AT)
(72) Erfinder: Guger, Christoph, 4533 Piberbach (AT); Edlinger, Günter, 8020 Graz (AT)
(74) Vertreter: Wildhack & Jellinek
(86) Internationale Anmeldenummer: PCT/AT2017/060056
(87) Internationale Veröffentlichungsnummer: WO 2017/152206

(56) Entgegenhaltungen:
- EP-A1- 2 886 049
- GB-A- 2 522 195
- US-A1- 2009 054 758
- US-A1- 2013 172 721

## Beschreibung

Die Erfindung betrifft eine Elektrodenhaube umfassend zumindest eine Elektrodenanordnung zum Anlegen an den Kopf eines Probanden gemäß dem Patentanspruch 1.

Weiters betrifft die Erfindung eine EEG-Messanordnung gemäß Patentanspruch 8.

Aus dem Stand der Technik ist eine große Anzahl unterschiedlicher Elektrodenhauben zum Anlegen an den Kopf eines Probanden bekannt. Die Schrift EP 2 886 049 A1 offenbart ein Kleidungsstück mit einer Elektrodenanordnung zur Messung von Biopotentialen gemäß des Oberbegriffs des Anspruchs 1. Allgemein besteht bei solchen Elektrodenhauben der Nachteil, dass Messsignale an einzelnen Elektroden unterschiedlichen Störungen unterliegen.

Die Erfindung stellt sich die Aufgabe, eine Elektrodenhaube zur Verfügung zu stellen, die gegenüber Störungen, die von elektromagnetischen Feldern oder Strukturen außerhalb der Elektrodenhaube ausgehen, weitestgehend unbeeinflusst ist. Die vorliegende Erfindung löst diese Aufgabe bei einer Elektrodenhaube der eingangs genannten Art mit den Merkmalen des Patentanspruchs 1.

Dabei ist vorgesehen, dass jede der Elektrodenanordnungen umfasst:
- eine Isolierschicht,
- zwei einander an der Isolierschicht gegenüberliegende Elektroden, nämlich eine erste, dem Kopf des Probanden zugewandte Messelektrode und eine dem Kopf des Probanden abgewandte Referenzelektrode, und
- einen an der Referenzelektrode anliegenden und mit dieser in elektrischem Kontakt stehenden leitfähiger Körper, der auf der dem Kopf des Probanden abgewandten Seite der Referenzelektrode angeordnet ist, und
- wobei die einzelnen leitfähigen Körper aller Elektrodenanordnungen elektrisch leitend miteinander verbunden sind.

Hierbei ist besonders vorteilhaft, dass durch den durchgängigen leitfähigen Körper elektromagnetische Einflüsse von außerhalb der Elektrodenhaube die Messsignale sämtlicher Elektroden gleichermaßen beeinflussen, sodass durch einfache Kompensation Signale erhalten werden können, die von den äußeren elektromagnetischen Einflüssen bzw. Störungen vollkommen unabhängig sind.

Besonders bevorzugt kann zum Anschluss an eine gemeinsame Systemmasse einer Messanordnung vorgesehen sein, dass eine Referenzelektrodenanordnung zum Anlegen an den Kopf des Probanden vorgesehen ist, die zumindest eine Elektrode aufweist, die dem Kopf des Probanden zugewandt ist und die elektrisch leitfähig mit den einzelnen leitfähigen Körpern der Elektrodenanordnungen verbunden ist.

In einer baulich besonders einfach ausgestalteten Ausführungsform der Erfindung ist vorgesehen, dass der leitfähige Körper der Referenzelektrodenanordnung mit den leitfähigen Körpern aller Elektrodenanordnungen elektrisch leitend verbunden ist.

Eine einfache Möglichkeit zur Schaffung eines durchgängigen leitfähigen Körpers sieht vor, dass der leitfähige Körper der oder jeder Elektrodenanordnung, gegebenenfalls der Referenzelektrodenanordnung, durch ein mit der Referenzelektrode und/oder mit der zweiten Elektrode in elektrischem Kontakt stehendes Fluid, insbesondere eine Kochsalzlösung, ausgebildet ist, und/oder dass der leitfähige Körper eine spezifische Leitfähigkeit von zwischen 2 mS/cm und 40mS/cm aufweist.

Hierbei kann zur modularen Ausbildung einzelner Elektrodenanordnungen vorgesehen sein, dass die einzelnen Elektrodenanordnungen und gegebenenfalls die Referenzelektrodenanordnung jeweils einen von mit der Referenzelektrode und/oder der zweiten Elektrode begrenzten Behälter aufweist, in dem das Fluid enthalten ist, und dass die einzelnen Behälter miteinander, insbesondere über Schlauchverbindungen, in Verbindung stehen und derart kommunizierende Gefäße bilden.

Um eine besonders einfache modulare Anpassung der Elektrodenhaube an unterschiedliche Kopfformen vorzusehen, kann vorgesehen sein, dass die Elektrodenhaube als schlauchförmiges Netz von miteinander über Schlauchverbindungen verbundenen Elektrodenanordnungen ausgebildet ist, wobei die einzelnen Behälter der Elektrodenanordnungen Anschlüsse zum, insbesondere reversiblen, Verbinden mit Schlauchverbindungen aufweisen.

Eine besonders vorteilhafte Kontaktierung des Kopfes des Probanden mit der Elektrodenhaube sieht vor, dass zumindest eine oder jede der Elektrodenanordnungen, und gegebenenfalls die Referenzelektrodenanordnung, eine von der Messelektrode oder der ersten Elektrode begrenzte und zum Kopf des Probanden hin offene Ausnehmung zur Aufnahme von leitfähigem Gel aufweist.

Eine vorteilhafte Messanordnung umfasst eine erfindungsgemäße Elektrodenhaube sowie eine Messeinrichtung zur Bestimmung der an den Elektroden anliegenden Spannungen. Besonders vorteilhaft kann zur Weiterverarbeitung der Signale vorgesehen sein, dass eine Verarbeitungseinheit vorgesehen ist, die diejenigen elektrischen Signale, die an der Messelektrode und der Referenzelektrode jeweils derselben Elektrodenanordnung anliegen, miteinander in Beziehung setzt, insbesondere die beiden Signale voneinander abzieht, und derart ein bereinigtes Signal für die Elektrodenanordnung erstellt.

Besonders einfach kann zur Schaffung eines Bezugspotentials vorgesehen sein, dass die zumindest eine Elektrode oder die beiden Elektroden der Referenzelektrodenanordnung mit einem Bezugspotential der Messeinrichtung verbunden sind.

Eine bevorzugte Ausführungsform der Erfindung wird anhand der folgenden Zeichnungsfiguren näher dargestellt.

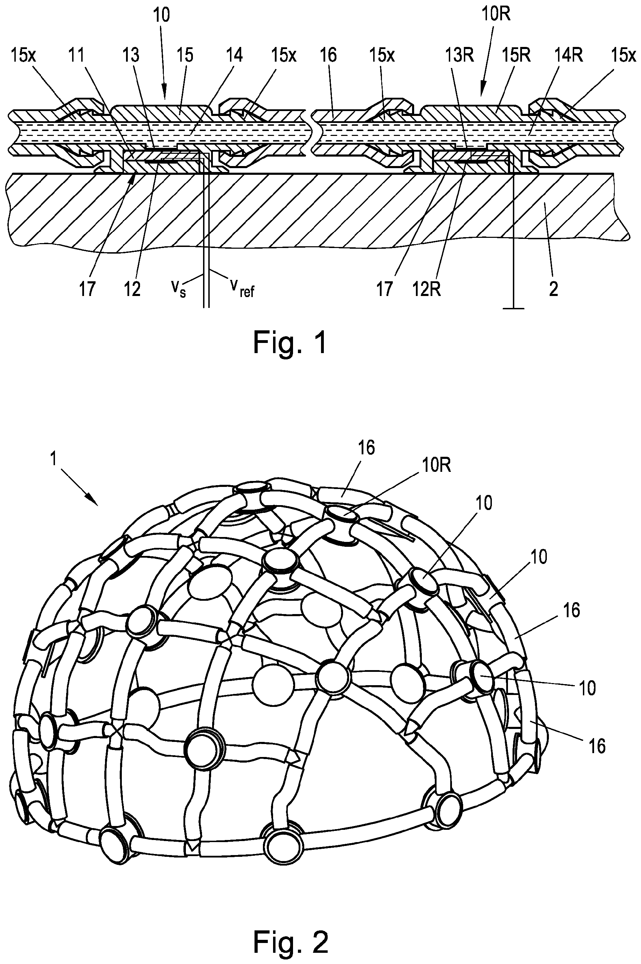

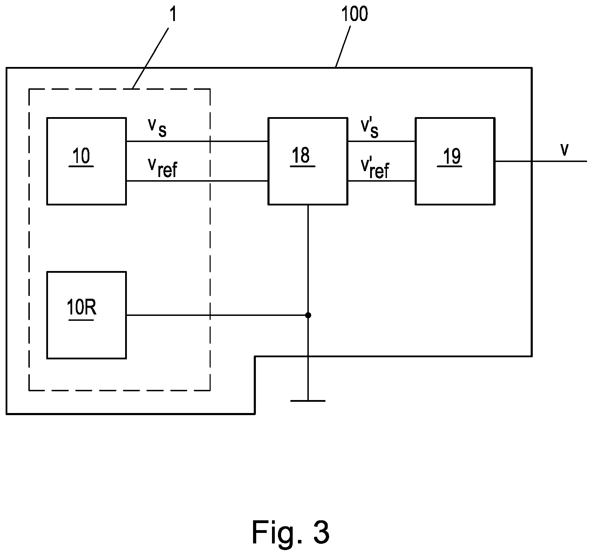

**Fig. 1** zeigt schematisch den Aufbau zweier Elektrodenanordnungen mit einem gemeinsamen leitfähigen Körper. **Fig. 2** zeigt den Aufbau einer Elektrodenhaube in ihrer Gesamtheit. In **Fig. 3** ist eine EEG-Messanordnung entsprechend einem Ausführungsbeispiel der Erfindung näher dargestellt.

In **Fig. 1** ist der Kopf eines Probanden 2 dargestellt, an den eine Elektrodenanordnung 10 sowie eine Referenzelektrodenanordnung 10R angeschlossen sind. Die erste Elektrodenanordnung 10 umfasst eine Isolierschicht 11 sowie zwei Elektroden 12, 13, die einander an der Isolierschicht 11 gegenüberliegen. Die erste der beiden gegenüberliegenden Elektroden 12 liegt an der dem Kopf des Probanden 2 zugewandten Seite. Die Elektrodenanordnung 10 weist eine Ausnehmung 17 zur Aufnahme von leitfähigem Gel auf, die von der ersten Elektrode 10 begrenzt ist und zum Kopf des Probanden 2 hin offen ist. In diese Ausnehmung 17 kann leitfähiges Gel eingefügt werden, sodass die erste Elektrode 12 mit dem Kopf des Probanden 2 in elektrischem Kontakt steht.

Weiters verfügt jede der Elektrodenanordnungen 10 jeweils über eine Referenzelektrode 13, die an derjenigen Seite der Isolierschicht 11 angeordnet ist, die der Messelektrode 12 gegenüberliegt. Die Referenzelektrode 13 steht mit einem leitfähigen Körper 14, der an der Referenzelektrode 13 anliegt, in elektrisch leitfähigem Kontakt. Der leitfähige Körper 14 ist auf der dem Kopf des Probanden 2 abgewandten Seite der Referenzelektrode 13 angeordnet. Die Elektrodenhaube 1 umfasst mehrere Elektrodenanordnungen 10 mit jeweils separater Isolierschicht 11 und jeweils separaten einander gegenüberliegenden Elektroden 12, 13. Für sämtliche oder für einen Teil der Elektrodenanordnungen 10 steht jeweils ein gemeinsamer leitfähiger Körper 14 zur Verfügung. Alternativ kann auch vorgesehen sein, dass für jede der Elektrodenanordnungen 10 jeweils ein separater leitfähiger Körper 14 besteht und sämtliche der leitfähigen Körper 14 der einzelnen Elektrodenanordnungen 10 miteinander elektrisch leitend verbunden sind.

Bei der in **Fig. 1** dargestellten Ausführungsform der Erfindung ist neben der Elektrodenanordnung 10 auch eine Referenzelektrodenanordnung 10R zum Anlegen an den Kopf des Probenden 2 vorgesehen. Die Referenzelektrodenanordnung 10R ist im wesentlichen so aufgebaut wie die Elektrodenanordnung 10 und weist insbesondere eine Elektrode 12R auf, die dem Kopf des Probanden 2 zugewandt ist. Insgesamt ist es jedoch lediglich erforderlich, dass eine einzige Elektrode 12R auf der dem Kopf des Probanden 2 zugewandten Seite vorgesehen ist, die, auf welche Art auch immer, leitfähig mit den einzelnen leitfähigen Körpern der Referenzelektrodenanordnung 10R verbunden ist. Besonders vorteilhaft kann eine solche Referenzelektrodenanordnung 10R realisiert werden, wenn diese ebenso wie die einzelnen Elektrodenanordnungen 10 ausgebildet ist, wobei die Referenzelektrodenanordnung 10R umfasst:
- eine Isolierschicht 11,
- zwei einander an der Isolierschicht 11 gegenüberliegende Elektroden 12R, 13R, nämlich eine erste, dem Kopf des Probanden 2 zugewandte erste Elektrode 12R und eine dem Kopf des Probanden 2 abgewandte zweite Elektrode 13R, wobei die erste und zweite Elektrode 13R miteinander elektrisch leitend verbunden sind,
- einen an der zweiten Elektrode 13R anliegenden und mit dieser in elektrischem Kontakt stehenden leitfähigen Körper 14R, der auf der dem Kopf des Probanden 2 abgewandten Seite der zweiten Elektrode 13R angeordnet ist.

Der leitfähige Körper 14R der Referenzelektrodenanordnung 10R ist mit den leitfähigen Körpern 14 der Elektrodenanordnungen 10 elektrisch leitend verbunden.

Wie auch in Hinblick auf Elektrodenanordnungen 10 eingangs dargestellt, besteht auch für die Referenzelektrodenanordnung 10R bevorzugt die Möglichkeit, dass eine von der ersten Elektrode 12R begrenzte und zum Kopf des Probanden 2 hin offene Ausnehmung 17 zur Aufnahme von leitfähigem Gel vorgesehen ist.

In den **Fig. 1 und 2** ist eine besonders bevorzugte Ausführungsform der Erfindung näher dargestellt, die über einen gemeinsamen leitfähigen Körper 14 verfügt, der durch ein mit den einzelnen Referenzelektroden 13 sowie gegebenenfalls der zweiten Elektrode 13R in Kontakt stehendem Fluid, insbesondere einer Kochsalzlösung ausgebildet ist. Der leitfähige Körper 14 weist in dieser bevorzugten Ausführungsform der Erfindung eine spezifische Leitfähigkeit zwischen 2 mS/cm und 40 mS/cm auf.

Wie in **Fig. 1** dargestellt, weist jede einzelne Elektrodenanordnung 10 sowie die Referenzelektrodenanordnung 10R jeweils einen von mit der Referenzelektrode 13 bzw. der zweiten Elektrode 13R begrenzten Behälter 15, 15R auf. In diesem Behälter ist das den leitfähigen Körper 14 bildende Fluid enthalten. Die einzelnen Behälter 15, 15R der Elektrodenanordnung 10 bzw. Referenzelektrodenanordnung 10R sind miteinander über Schlauchverbindungen 16 verbunden und bilden derart ein kommunizierendes Gefäß. Wie in **Fig. 2** dargestellt, weist jede der Elektrodenanordnungen 10 bzw. der Referenzelektrodenanordnungen 10R jeweils eine Anzahl von Anschlüssen 15X für Schlauchverbindungen 16 auf. Die einzelnen Elektrodenanordnungen des vorliegenden Ausführungsbeispiels weisen jeweils vier Schlauchverbindungen 15X auf. Die Anzahl der Schlauchverbindungen 15X kann aber auch variieren beispielsweise zwischen zwei und zehn liegen. Die Elektrodenhaube 1 ist als schlauchförmiges Netz von miteinander in Schlauchverbindungen 16 verbundenen Elektrodenanordnungen 10, 10R ausgebildet. In dieser bevorzugten Ausführungsform der Erfindung können die Schlauchverbindungen 16 reversibel von den Anschlüssen 15X gelöst werden. Wie in **Fig. 2** dargestellt, besteht die Möglichkeit, dass die einzelnen Schlauchverbindungen 16R neben den Behältern 15 der Elektrodenanordnungen 10 auch in T-Stücke münden. Darüber hinaus besteht auch die Möglichkeit, dass einzelne der Schlauchverbindungen 16 mit einem externen Zuflussschlauch und einem externen Abflussschlauch verbunden sind, über den Fluid in die Elektrodenhaube 1 bzw. in das Schlauchnetzwerk gepumpt werden kann bzw. über den das Fluid aus dem Schlauchnetzwerk bzw. aus der Elektrodenhaube 1 abgepumpt werden kann.

In **Fig. 3** ist eine EEG-Messanordnung 100 entsprechend einem Ausführungsbeispiel der Erfindung näher dargestellt. Diese EEG-Messanordnung 100 umfasst eine Elektrodenhaube 1 wie in **Fig. 1 und 2** dargestellt sowie eine Messeinrichtung 18 zur Messung von elektrischen Spannungen bzw. Spannungssignalen. Die einzelnen Elektroden 12, 13 der Elektrodenanordnungen 10 sind elektrisch leitfähig mit der Messeinrichtung 18 verbunden. Auch die Elektroden 12R der Referenzelektrodenanordnung 10R sind mit der elektrischen Messeinrichtung 18 verbunden. Im konkreten Fall sind die beiden Elektroden 12R, 13R der Referenzelektrodenanordnung 10R mit dem Bezugspotential der Messeinrichtung 18 verbunden. Die Messeinrichtung 18 ermittelt laufend die einzelnen an den Elektroden 12, 13 anliegenden Spannungen und stellt derart Signale V_{S}, V_{ref} zur Verfügung. Die EEG-Messanordnung 100 umfasst eine Verarbeitungseinheit 19, die der elektrischen Messanordnung nachgeschaltet ist. Diese Verarbeitungseinheit 19 setzt die einzelnen von der Messeinrichtung 18 aufgenommenen elektrischen Signale V_{S}, V_{ref}, die an der Messelektrode 12 und der Referenzelektrode 13 jeweils derselben Elektrodenanordnung 10 anliegen, miteinander in Bezug. Bei einer bevorzugten Ausführungsform der Erfindung zieht sie die beiden Signale voneinander ab und bildet derart ein bereinigtes Signal V, das sie an ihrem Ausgang zur Verfügung stellt.

## Patentansprüche

1. Elektrodenhaube (1) umfassend zumindest eine Elektrodenanordnung (10) zum Anlegen an den Kopf eines Probanden (2),
- wobei jede der Elektrodenanordnungen (10) umfasst:
- eine Isolierschicht (11),
- zwei einander an der Isolierschicht (11) gegenüberliegende Elektroden (12, 13), nämlich eine erste, dem Kopf des Probanden (2) zugewandte Messelektrode (12) und eine dem Kopf des Probanden (2) abgewandte Referenzelektrode (13), und
- einen an der Referenzelektrode (13) anliegenden und mit dieser in elektrischem Kontakt stehenden leitfähiger Körper (14), der auf der dem Kopf des Probanden (2) abgewandten Seite der Referenzelektrode (13) angeordnet ist, und
- wobei die einzelnen leitfähigen Körper (14) aller Elektrodenanordnungen (10) elektrisch leitend miteinander verbunden sind,
**dadurch gekennzeichnet, dass** der leitfähige Körper (14) zumindest einer oder jeder Elektrodenanordnung (10) durch ein mit der Referenzelektrode (13) in elektrischem Kontakt stehendes Fluid, insbesondere eine Kochsalzlösung, ausgebildet ist.

2. Elektrodenhaube (1) nach Anspruch 1, **dadurch gekennzeichnet, dass** eine Referenzelektrodenanordnung (10R) zum Anlegen an den Kopf des Probanden (2) vorgesehen ist, die zumindest eine Elektrode aufweist, die dem Kopf des Probanden (2) zugewandt ist und die elektrisch leitfähig mit den einzelnen leitfähigen Körpern (14) der Elektrodenanordnungen (10) verbunden ist.

3. Elektrodenhaube (1) nach Anspruch 2, **dadurch gekennzeichnet, dass** die Referenzelektrodenanordnung (10R) umfasst:
- eine Isolierschicht (11R),
- zwei einander an der Isolierschicht (11R) gegenüberliegende Elektroden (12R, 13R), nämlich eine erste, dem Kopf des Probanden (2) zugewandte erste Elektrode (12R) und eine dem Kopf des Probanden (2) abgewandte zweite Elektrode (13R), wobei die erste und zweite Elektrode (12R, 13R) miteinander elektrisch leitend verbunden sind,
- einen an der zweiten Elektrode (13) anliegenden und mit dieser in elektrischem Kontakt stehenden leitfähigen Körper (14R), der auf der dem Kopf des Probanden (2) abgewandten Seite der zweiten Elektrode (13R) angeordnet ist,
wobei der leitfähige Körper (14R) der Referenzelektrodenanordnung (10R) mit den leitfähigen Körpern (14) aller Elektrodenanordnungen (10) elektrisch leitend verbunden ist.

4. Elektrodenhaube (1) nach Anspruch 3, **dadurch gekennzeichnet, dass** der leitfähige Körper (14R) der Referenzelektrodenanordnung (10R) durch ein mit der zweiten Elektrode (13R) in elektrischem Kontakt stehendes Fluid, insbesondere eine Kochsalzlösung, ausgebildet ist.

5. Elektrodenhaube (1) nach einem der vorangehenden Ansprüche, **dadurch gekennzeichnet,**
- **dass** die einzelnen Elektrodenanordnungen (10) und gegebenenfalls die Referenzelektrodenanordnung (10R) jeweils einen von mit der Referenzelektrode (13) und/oder der zweiten Elektrode (13R) begrenzten Behälter (15, 15R) aufweist, in dem das Fluid enthalten ist, und
- **dass** die einzelnen Behälter (15) miteinander, insbesondere über Schlauchverbindungen (16), in Verbindung stehen und derart kommunizierende Gefäße bilden.

6. Elektrodenhaube (1) nach Anspruch 5, **dadurch gekennzeichnet, dass** die Elektrodenhaube (1) als schlauchförmiges Netz von miteinander über Schlauchverbindungen (16) verbundenen Elektrodenanordnungen (10, 10R) ausgebildet ist, wobei die einzelnen Behälter (15) der Elektrodenanordnungen (10, 10R) Anschlüsse (15x) zum, insbesondere reversiblen, Verbinden mit Schlauchverbindungen (16) aufweisen.

7. Elektrodenhaube (1) nach einem der vorangehenden Ansprüche, **dadurch gekennzeichnet, dass** zumindest eine oder jede der Elektrodenanordnungen (10), und gegebenenfalls die Referenzelektrodenanordnung (10R), eine von der Messelektrode (12) oder der ersten Elektrode (12R) begrenzte und zum Kopf des Probanden (2) hin offene Ausnehmung (17) zur Aufnahme von leitfähigem Gel aufweist.

8. Elektrodenhaube (1) nach einem der vorangehenden Ansprüche, **dadurch gekennzeichnet, dass** der leitfähige Körper (14, 14R) eine spezifische Leitfähigkeit von zwischen 2 mS/cm und 40 mS/cm aufweist.

9. EEG-Messanordnung (100) umfassend eine Elektrodenhaube (1) nach einem der vorangehenden Ansprüche und eine Messeinrichtung (18), **dadurch gekennzeichnet, dass** die einzelnen Elektroden (12, 13) der Elektrodenanordnungen (10), und gegebenenfalls die zumindest eine Elektrode (12R) der Referenzelektrodenanordnung (10R), elektrisch leitfähig mit der elektrischen Messeinrichtung (18), insbesondere zur Messung der an den einzelnen Elektroden (12, 13) anliegenden Spannungen und/oder Signalen (V_{S}, V_{ref}), verbunden sind.

10. EEG-Messanordnung (100) nach Anspruch 9, **dadurch gekennzeichnet, dass** eine Verarbeitungseinheit (19) vorgesehen ist, die diejenigen elektrischen Signale (V_{S}, V_{ref}), die an der Messelektrode (12) und der Referenzelektrode (13) jeweils derselben Elektrodenanordnung (10) anliegen, miteinander in Beziehung setzt, insbesondere die beiden Signale (V_{S}, V_{ref}) voneinander abzieht, und derart ein bereinigtes Signal (V) für die Elektrodenanordnung (10) erstellt.

11. EEG-Messanordnung (100) nach Anspruch 9 oder 10, **dadurch gekennzeichnet, dass** die zumindest eine Elektrode (12R) oder die beiden Elektroden (12R, 13R) der Referenzelektrodenanordnung (10R) mit einem Bezugspotential der Messeinrichtung (18) verbunden sind.

## Claims

1. Electrode cap (1) comprising at least one electrode assembly (10) for placing on the head of a test subject (2),
- wherein each of the electrode assemblies (10) comprises:
- an insulating layer (11),
- two electrodes (12, 13) lying opposite each other on the insulating layer (11), namely a first measurement electrode (12) facing the head of the test subject (2) and a reference electrode (13) facing away from the head of the test subject (2), and
- a conductive body (14) which abuts against the reference electrode (13) and electrically contacts same and which is arranged on the side of the reference electrode (13) facing away from the head of the test subject (2); and
- wherein the individual conductive bodies (14) of all of the electrode assemblies (10) are connected to one another in an electrically conductive manner,
**characterised in that** the conductive body (14) of at least one or each of the electrode assemblies (10) is formed by a fluid in electrical contact with the reference electrode (13), in particular a saline solution.

2. Electrode cap (1) according to claim 1, **characterised in that** a reference electrode assembly (10R) is provided for placing on the head of the test subject (2), which has at least one electrode that is facing the head of the test subject (2) and which is connected in an electrically conductive manner with the individual conductive bodies (14) of the electrode assemblies (10).

3. Electrode cap (1) according to claim 2, **characterised in that** the reference electrode assembly (10R) comprises:
- an insulating layer (11R),
- two electrodes (12R, 13R) lying opposite each other on the insulating layer (11R), namely a first electrode (12R) facing the head of the test subject (2) and a second electrode (13R) facing away from the head of the test subject (2), wherein the first and second electrode (12R, 13R) are connected to each other in an electrically conductive manner,
- a conductive body (14R) which abuts against the second electrode (13) and electrically contacts same and which is arranged on the side of the second electrode (13R) facing away from the head of the test subject (2); and
wherein the conductive body (14R) of the reference electrode assembly (10R) is connected to the conductive bodies (14) of all the electrode assemblies (10) in an electrically conductive manner.

4. Electrode cap (1) according to claim 3, **characterised in that** the conductive body (14R) of the reference electrode assembly (10R) is formed by a fluid in electrical contact with the second electrode (13R), in particular a saline solution.

5. Electrode cap (1) according to any one of the preceding claims, **characterised in that**
- the individual electrode assemblies (10) and where appropriate the reference electrode assembly (10R) each have a container (15, 15R) delimited by the reference electrode (13) and/or the second electrode (13R), in which the fluid is contained, and
- **in that** the individual containers (15) are connected to each other, in particular via tube connections (16), and thus form communicating vessels.

6. Electrode cap (1) according to claim 5, **characterised in that** the electrode cap (1) is formed as a tubular network of electrode assemblies (10, 10R) connected together by tube connections (16), wherein the individual containers (15) of the electrode assemblies (10, 10R) have connections (15x) for, in particular reversible, connecting to tube connections (16).

7. Electrode cap (1) according to anyone of the preceding claims, **characterised in that** at least one or each of the electrode assemblies (10), and where appropriate the reference electrode assembly (10R), has a recess (17) delimited by the measurement electrode (12) or the first electrode (12R) and is open towards the head of the test subject (2) for receiving conductive gel.

8. Electrode cap (1) according to anyone of the preceding claims, **characterised in that** the conductive body (14, 14R) has a specific conductivity of between 2 mS/cm and 40 mS/cm.

9. EEG measurement assembly (100) comprising an electrode cap (1) according to any of the preceding claims and a measurement unit (18), **characterised in that** the individual electrodes (12, 13) of the electrode assemblies (10), and where appropriate the at least one electrode (12R) of the reference electrode assembly (10R), are connected to the electrical measurement unit (18) in an electrically conductive manner, in particular for measuring the voltages and/or signals (V_{S}, V_{ref}), present on the individual electrodes (12, 13).

10. EEG measurement assembly (100) according to claim 9, **characterised in that** a processing unit (19) is provided which relates to each other the electrical signals (V_{S}, V_{ref}) which are present on the measurement electrode (12) and the reference electrode (13) of the same electrode assembly (10), in particular subtracts the two signals (V_{S}, V_{ref}) from each other, and in this way establishes a cleaned signal (V) for the electrode assembly (10).

11. EEG measurement assembly (100) according to claim 9 or 10, **characterised in that** the at least one electrode (12R) for the two electrodes (12R, 13R) of the reference electrode assembly (10R) are connected to a reference potential of the measurement unit (18).

## Revendications

1. Casque à électrodes (1) comprenant au moins un ensemble d'électrodes (10) destiné à être appliqué sur la tête d'un sujet (2),
- dans lequel chacun des ensembles d'électrodes (10) comprend :
- une couche isolante (11),
- deux électrodes (12, 13) placées en vis-à-vis sur la couche isolante (11), à savoir une première électrode de mesure (12) tournée vers la tête du sujet (2) et une électrode de référence (13) tournée à l'opposé de la tête du sujet (2), et
- un corps conducteur (14) appliqué sur l'électrode de référence (13) et en contact électrique avec celle-ci, qui est agencé sur le côté de l'électrode de référence (13) opposé à la tête du sujet, et
- dans lequel les corps conducteurs individuels (14) de tous les ensembles d'électrodes (10) sont connectés les uns aux autres d'une manière électriquement conductrice,
**caractérisé en ce que** le corps conducteur (14) d'au moins un ou de chaque ensemble d'électrodes (10) est formé par un fluide en contact électrique avec l'électrode de référence (13), en particulier une solution saline.

2. Casque à électrodes (1) selon la revendication 1, **caractérisé en ce qu'**un ensemble d'électrodes de référence (10R) destiné à être appliqué sur la tête du sujet à tester (2) est prévu, lequel présente au moins une électrode qui fait face à la tête du sujet (2) et qui est connectée d'une manière électriquement conductrice aux corps conducteurs individuels (14) des ensembles d'électrodes (10).

3. Casque à électrodes (1) selon la revendication 2, **caractérisé en ce que** l'ensemble d'électrodes de référence (10R) présente :
- une couche isolante (11R),
- deux électrodes (12R, 13R) placées en vis-à-vis sur la couche isolante (11R), à savoir une première électrode (12R) tournée vers la tête du sujet (2) et une seconde électrode (13R) tournée à l'opposé de la tête du sujet (2), les première et seconde électrodes (12R, 13R) étant connectées l'une à l'autre d'un manière électriquement conductrice,
- un corps conducteur (14R) appliqué sur la seconde électrode (13) et en contact électrique avec celle-ci et qui est agencé sur le côté de la seconde électrode (13R) tourné à l'opposé de la tête du sujet (2),
dans lequel le corps conducteur (14R) de l'ensemble d'électrodes de référence (10R) est connecté d'une manière électriquement conductrice aux corps conducteurs (14) de tous les ensembles d'électrodes (10).

4. Casque à électrodes (1) selon la revendication 3, **caractérisé en ce que** le corps conducteur (14R) de l'ensemble d'électrodes de référence (10R) est formé par un fluide qui est en contact électrique avec la seconde électrode (13R), en particulier une solution saline.

5. Casque à électrodes (1) selon l'une des revendications précédentes, **caractérisé en ce que**
- les ensembles d'électrodes individuels (10) et facultativement l'ensemble d'électrodes de référence (10R) présentent chacun un contenant (15, 15R), qui est délimité par l'électrode de référence (13) et/ou la seconde électrode (13R), dans lequel le fluide est contenu, et
- les contenants individuels (15) sont reliés les uns aux autres, en particulier via des raccords de tuyaux (16), et forment des réceptacles qui communiquent de cette manière.

6. Casque à électrodes (1) selon la revendication 5, **caractérisé en ce que** le casque à électrodes (1) est conçu sous la forme d'un réseau de forme tubulaire d'ensembles d'électrodes (10, 10R) reliés les uns aux autres via des raccords de tuyaux (16), dans lequel les contenants individuels (15) des ensembles d'électrodes (10, 10R) présentent des raccords (15x) pour un raccordement, en particulier réversible, avec des raccords de tuyaux (16).

7. Casque à électrodes (1) selon l'une des revendications précédentes, **caractérisé en ce qu'**au moins un ou chacun des ensembles d'électrodes (10), et facultativement l'ensemble d'électrodes de référence (10R), présente un évidement (17) délimité par l'électrode de mesure (12) ou la première électrode (12R) et ouvert vers la tête du sujet (2) pour recevoir du gel conducteur.

8. Casque à électrodes (1) selon l'une des revendications précédentes, **caractérisé en ce que** le corps conducteur (14, 14R) présente une conductivité spécifique comprise entre 2 mS/cm et 40 mS/cm.

9. Ensemble de mesure d'EEG (100) comprenant un casque à électrodes (1) selon l'une des revendications précédentes et un dispositif de mesure (18), **caractérisé en ce que** les électrodes individuelles (12, 13) des ensembles d'électrodes (10), et facultativement la au moins une électrode (12R) de l'ensemble d'électrodes de référence (10R), sont connectées de manière électriquement conductrice au dispositif de mesure électrique (18), en particulier pour mesurer les tensions et/ou les signaux (Vₛ, V_{ref}) appliqué(e)s aux électrodes individuelles (12, 13).

10. Ensemble de mesure d'EEG (100) selon la revendication 9, **caractérisé en ce qu'**une unité de traitement (19) est prévue, qui met en relation les signaux électriques (Vₛ, V_{ref}) respectifs qui sont appliqués à l'électrode de mesure (12) et à l'électrode de référence (13) du même ensemble d'électrodes (10), en particulier soustrait les deux signaux (Vₛ, V_{ref}) l'un de l'autre, et crée ainsi un signal nettoyé (V) pour l'ensemble d'électrodes (10).

11. Ensemble de mesure d'EEG (100) selon la revendication 9 ou 10, **caractérisé en ce que** la au moins une électrode (12R) ou les deux électrodes (12R, 13R) de l'ensemble d'électrodes de référence (10R) sont connectées avec un potentiel de référence du dispositif de mesure (18).
